# EUROPEAN PATENT SPECIFICATION

(11) **EP 1 613 294 B1**
(45) Date of publication and mention of the grant of the patent: **11.03.2009**
(21) Application number: 04726735.6
(22) Date of filing: 09.04.2004
(51) Int. Cl.: A61K 38/05, C07K 5/06, A61P 17/18

(54) **PEPTIDES AND DERIVATIVES THEREOF AS INHIBITORS OF OXIDATIVE DAMAGE OF THE SKIN**
ZUSAMMENSETZUNGEN UND VERWENDUNG VON PEPTIDEN UND DEREN DERIVATE ALS HEMMER DER OXIDATIVEN SCHÄDIGUNG DER HAUT
PEPTIDES ET DERIVES DE CEUX-CI SERVANT D'INHIBITEURS DES LESIONS OXYDATIVES DE LA PEAU

(30) Priority: 11.04.2003 IT MI20030755
(43) Date of publication of application: 11.01.2006
(73) Proprietor: Bottega Verde S.r.l., 53026 Pienza (SI) (IT)
(72) Inventor: GHIRARDINI, Adriano, I-48100 Ravenna (IT); CERONI, Milla, I-13836 Cossato (BI) (IT)
(74) Representative: Pipparelli, Claudio
(86) International application number: PCT/IT2004/000199
(87) International publication number: WO 2004/089354

(56) References cited:
- US-A- 4 569 794
- US-A1- 2002 058 608
- GUTIERREZ-CORREA JOSE ET AL: "Phenothiazine radicals inactivate Trypanosoma cruzi dihydrolipoamide dehydrogenase: Enzyme protection by radical scavengers." FREE RADICAL RESEARCH, vol. 37, no. 3, March 2003 (2003-03), pages 281-291, XP009037694 ISSN: 1071-5762
- SUETSUNA KUNIO ET AL: "Antihypertensive effect of wakame peptide on blood pressure in spontaneously hypertensive rats." JOURNAL OF NATIONAL FISHERIES UNIVERSITY, vol. 51, no. 4, March 2003 (2003-03), pages 141-146, XP001184011 ISSN: 0370-9361
- PATENT ABSTRACTS OF JAPAN vol. 018, no. 656 (C-1286), 13 December 1994 (1994-12-13) & JP 06 256387 A (YOKO SUETSUNA), 13 September 1994 (1994-09-13)
- PATENT ABSTRACTS OF JAPAN vol. 017, no. 577 (C-1122), 20 October 1993 (1993-10-20) -& JP 05 170636 A (NARISU KESHOHIN:KK), 9 July 1993 (1993-07-09)
- PARAC, TATJANA N. ET AL: "New Selectivity and Turnover in Peptide Hydrolysis by Metal Complexes. A Palladium(II) Aqua Complex Catalyzes Cleavage of Peptides Next to the Histidine Residue" JOURNAL OF THE AMERICAN CHEMICAL SOCIETY , CODEN: JACSAT; , vol. 118, no. 1, 1996, pages 51-58, XP002302140 ISSN: 0002-7863
- RADOMSKA B ET AL: "TRANSITION-METAL COMPLEXES OF HISTIDYLPHENYLALANINE AND HISTIDYLTYROSINE" JOURNAL OF CHEMICAL RESEARCH (SYNOPSES), no. 5, 1987, pages 156-157, XP009037686 ISSN: 0308-2342
- BURCHAM PHILIP C ET AL: "Aldehyde-sequestering drugs: tools for studying protein damage by lipid peroxidation products." TOXICOLOGY. 27 DEC 2002, vol. 181-182, 27 December 2002 (2002-12-27), pages 229-236, XP002302141 ISSN: 0300-483X

## Description

The present invention relates to the utilization of compounds of peptidic nature as inhibitors and preventive agents of oxidative-induced degradation phenomena, as well as the use of the above compounds in the inhibition/prevention of degenerative/degradation processes induced by the reactive intermediate such as aldehydes and keto-aldehydes arising from the oxidation of endogenous and exogenous lipids, sugars and proteins.

The present invention also relates to the preparation of functionalised derivatives of the said peptidic compounds and their use for the same purpose, as well as to the process for their preparation and their use, alone or in combination, for cosmetic, pharmaceutical and nutritional purposes.

### Background of the invention

It is well documented that oxidative stress plays an important role in the onset and propagation of many chronic degenerative pathologies [Esterbauer H., Schaur R.J., Zollner H.; Free Rad. Biol. Med., ,11, 81-128, 1991], and that the peroxidation of endogenous lipids, sugars and proteins, due to a variety of oxidative stress (chemical, physical or biological), gives rise to a burst of carbonylic species among which 4-hydroxy-2-nonenal, acrolein, crotonaldehyde, nonenal, glyoxal, methyl glyoxal and malonyl-di-aldehyde. These compounds are highly reactive and able to interact with the nucleophilic sites of proteins (nucleic acids etc), trough a Michael addition reaction and formation of Schiff bases, forming intra- and intermolecular cross links, responsible for the destructuration of the macromolecular architecture of the biological target, loss of functionality, and ultimately for cytotoxic and mutagenic effects [ Poli G, Schaur J, IUMB Life, 50,1-7, 2000]. Furthermore, aldehydes and keto-aldehydes are formed in human body due to oxidative stress, and these degradation products contribute to different patho-physiological processes: cardiovascular diseases including atherosclerosis and long-term complications of diabetes, muscular dystrophy, rheumatoid arthritis, actinic elastosis, aging and neurodegenerative diseases (e.g., Alzheimer's disease, Parkinson's disease, and cerebral ischemia) [Uchida K, Free Radic Biol Med, 28(12):1685-96, 2000; Picklo MJ, Montine TJ, Amarnath V, et al. Toxicol Appl Pharmacol, 184(3):187-97, 2002]. In particular, human skin is continuously exposed to oxidative stress of different nature i.e physical (UVA, UVB, VIS, IR Radiations, heat, cold, dryness, osmotic shock), chemical (ozone and nitrogen dioxide, etc.), and to the polluting action of transition metals, which catalyze free radical reactions [Wlaschek M, Tantcheva-Poor I, Naderi L, Ma W, Schneider LA, Razi-Wolf Z, Schuller J, Scharffetter-Kochanek K., J Photochem Photobiol B;63(1-3):41-51, 2001]. These oxidative processes promote the formation in the skin of reactive carbonyl compounds, which are responsible for skin aging and photo-aging and other pathological diseases of the epidermal and dermal layers, for example, actinic elastosis [Tanaka N, Tajima S, Ishibashi A, Uchida K, Shigematsu T. Arch Dermatol Res, 293(7):363-7, 2001]. Furthermore reactive aldehydes and keto-aldehydes present in the atmosphere as pollutants, are able to directly attack the surface and deeper layers of the skin. [Thiele JJ, Traber MG, Re R, Espuno N, Yan LJ, Cross CE, Packer L. FEBS Lett, 422(3):403-6, 1998]. Hence skin aging is the consequence of several factors among which :loss of natural antioxidant pool, free radical insult, carbonyl stress exposure, co-oxidation of nucleophilic cellular constituents, decrease of resistance and regenerative properties of the skin.

In the same way, the oxidation process of lipids, sugars and proteins and the relative formation of aldehydes and keto-aldehydes, is a degradative phenomenon, which can occur in raw materials, intermediate or finished products, used, for example, in cosmetic, food and pharmaceutical industry. Seed oils may contain a significant amount of saturated and unsaturated aldehydes, such as hexanal, hexa-dienal, acrolein and these can be mutagenic after ingestion [Grootveld M, Atherton MD, Sheerin AN, Hawkes J, Blake DR, Richens TE, Silwood CJ, Lynch E, Claxson AW. J Clin Invest, 101(6): 1210-8, 1998; Indart A, Viana M, Grootveld MC, Silwood CJ, Sanchez-Vera I, Bonet B. Free Radic Res, 36(10):1051-8, 2002]. Moreover, the degradation of fatty constituents, present in cosmetic formulations, into aldehydic byproducts is a problem well-known to the cosmetic chemist and their presence in the finished products, due to an uncorrected /inadequate preservation leads to an unpleasant smell, and can give rise to severe toxicological effects when the product is applied to the skin.

On the basis of the above evidences, the development of multi-functional molecules able not only to inhibit the free radical mediated oxidative process, but also to neutralise/entrap the reactive carbonyl products through a mechanism of quenching/detoxification, is of great relevance for cosmetic, food and pharmaceutical purposes.

The Applicant has now found that specific di-peptides are able to play this double role, acting: 1) as antioxidant agents, through a free radical scavenging mechanism; 2) as aldehydes and keto-aldehydes quenchers, to be used as such and/or in suitable formulations, for delaying, preventing/inhibiting the effects of the oxidative damage. The first object of the present invention relates to the use of dipeptides selected from H-His-Tyr-OH and/or H-Tyr-His-OH and/or N-acyl derivatives of the Tyr part of H-Tyr-His-OH, said acyl group having the formula -CO-(CH₂-)ₙCH₃ wherein n is from 0 to 18, for the manufacture of a medicament or a cosmetic product for the inhibition/prevention of the oxidative damage of the skin, particularly for the damage of the skin due to intrinsic- or photo- aging of the skin or due to physical (UV, vis IR radiations), thermal and mechanical stress of the skin.
The medicament or the cosmetic product is formulated as solution, surfactants containing suspension, gel, aqueous paste, gel emulsion, o/w emulsion, w/o emulsion, anhydrous lipidic system.
Dipeptide H-His-Tyr-OH has the following structure A: 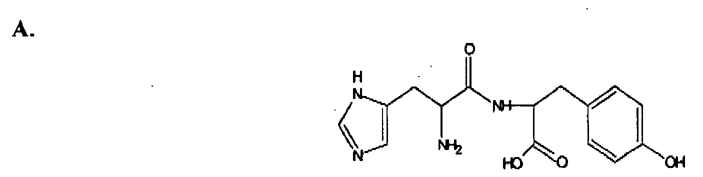 Dipeptide H-Tyr-His-OH has the following structure B

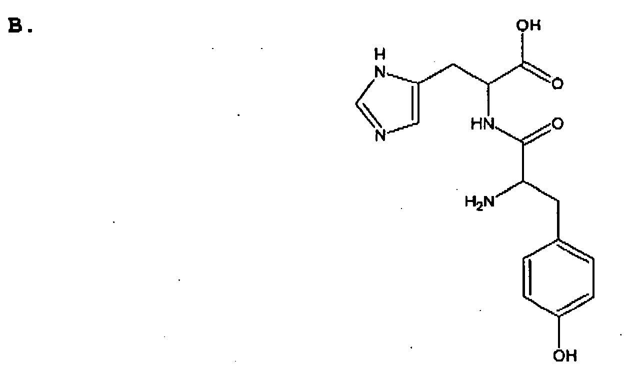

The second object of the present invention are N-acyl compounds of the Tyr part of H-Tyr-His-OH, said acyl group having the formula -CO-(CH₂-)ₙCH₃ wherein n is from 0 to 18, particularly n =14.

The biological activity of the dipeptides has been determined by the Applicant, according to the following procedures. This represents the first objective of the present invention.

### 1. Quenching activity of α,β-unsaturated aldehydes by H-His-Tyr-OH and H-Tyr-His-OH

4-hydroxy-trans-nonenal (HNE) was used throughout the study, as the prototype of α,β-unsaturated aldehydes since it represents the main oxidative degradation product of ω-6 polyunsaturated fatty acids, such as arachidonic and linoleic acids, and it is formed in large amount in the skin under different patho-physiological conditions, such as intrinsic and photo-induced skin aging [Tanaka N, Arch Dermatol Res, 293, 363-367, 2001].

### 1.1 Assays in homogeneous phase

A homogeneous phase model was first used to evaluate the ability of H-His-Tyr-OH and H-Tyr-His-OH to react and deactivate the cytotoxic agent HNE (*quenching* activity). In particular, the di-peptides were incubated at 37°C in a phosphate buffer, 10 mM pH 7.4, in the presence of 200 µM HNE. HNE consumption was determined after 1 hour and 24 hours of incubation by HPLC analysis, under the following experimental conditions: HP1050 (Hewlett Packard, Milano, Italy) equipped with a quaternary pump, self-sampling, on-line degasser, UV/VIS photodiode detector. The separations were performed by means of a Supelco LC18 column (25 cm x 4.6 mm i.d.; particle size 5 µm) and an isocratic elution system: 60% of phase A (H₂O/CH₃CN/HCOOH 90/1.0/0.] v/v/v) and 40% of phase B (H₂O/CH₃CN 10/90 v/v/v). The flow rate was maintained at 1 ml/min and the length of the revealing wave was fixed at 223 nm.

The results summarized in Table 1, expressed as a % of residual HNE, demonstrate that H-His-Tyr-OH and H-Tyr-His-OH are able to entrap the cytotoxic aldehyde, with a greater potency than Amino-guanidine, the well known *quencher* of α,β-unsaturated aldehydes, used as reference molecule [Burcham PC, Toxicology 181-182, 229-236, 2002; Al-Abed Y, Chem Res Toxicol 10, 875-879, 1997].

**Table 1**

| | **RESIDUAL HNE (%)** | |
|---|---|---|
| **Substance** | **1 hr of incubation** | **24 hrs of incubation** |
| H-His-Tyr-OH | 80.16 ± 7.57 | 15.41 ± 0.49 |
| H-Tyr-His-OH | 85.31 ± 6.29 | 20.02 ± 4.09 |
| Amino guanidine | 95.31 ± 0.08 | 72.04 ± 3.97 |

The values are expressed as the average ± S.D of 4 determinations.

The quenching mechanism was established by characterization of the reaction products by mass spectrometry using an Ion Trap mass spectrometer (Thermo Finnigan LCQ Advantage, Thermoquest, Milan, Italy) equipped with an Electro-spray (ESI) ionization source. The capillary temperature was maintained at 250°C and the ionization voltage at 5 kV; nitrogen at a flow rate of 0.5 1/min was used as nebulizing gas. The reaction mixture containing the di-peptide (2 mM) and HNE (200 µM), after incubation at 37°C for 24 hours, was 1:5 diluted with a mix of H₂O:CH₃CN (70:30) and introduced into the spectrometer by means of a Harvard syringe, at an infusion rate of 5 µl/min. The spectra were recorded in negative and positive ion modes, at a scanning rate of 0.5 scan/sec and within a mass range of between 100 and 1000 m/z. The instrumental parameters were optimised using LCQ-Xcalibur software.

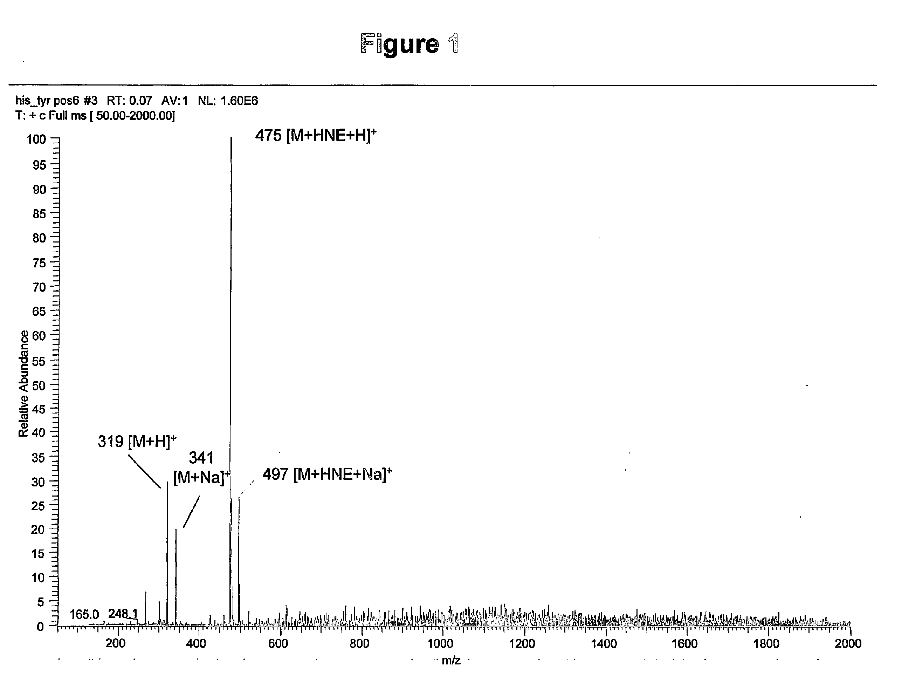

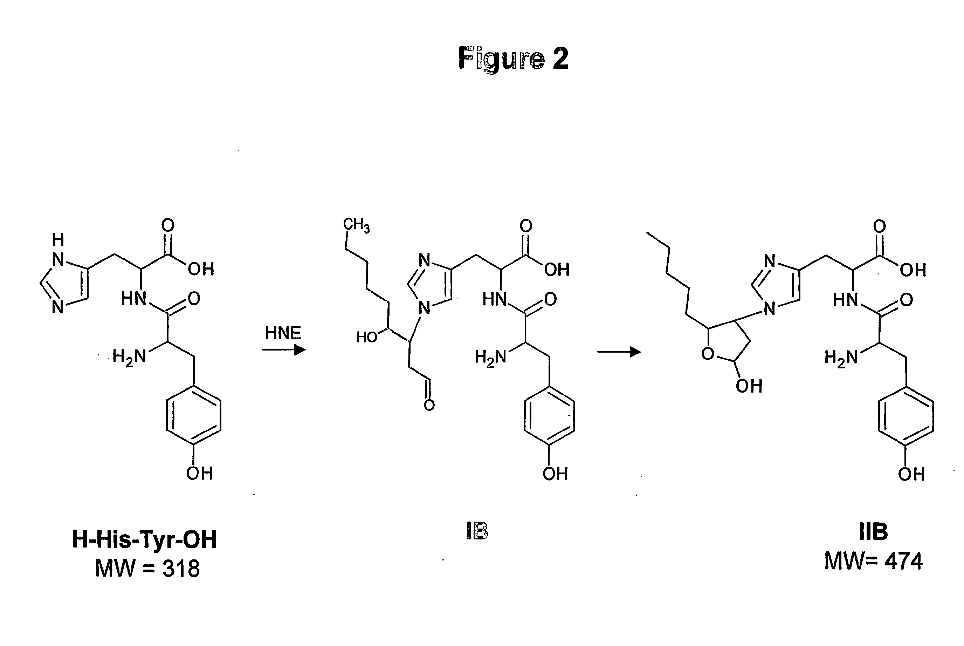

Figure 1 shows the mass spectrum (positive ion mode) of the reaction mixture containing H-His-Tyr-OH and HNE. The mass spectrum is characterized by the protonated molecular ion at m/z 319 relative to unreacted di-peptide substrate, and by the ions at m/z 475 and m/z 497, which can be attributed to the molecular ion and to the sodium adduct of the reaction product. On the basis of the molecular weight (474 Daltons), confirmed by the deprotonated molecular anion at m/z 473 (negative-ion mode), and of the fragmentation pattern obtained by collision of the molecular ion at 475 m/z (positive ion), the structure **HB** (Figure 2) was attributed to the Michael addition product. Hence the reaction mechanism, (Figure 2) involves Michael addition between the nucleophilic imidazole nitrogen of the histidine residue, and the electrophilic carbon centre (C3) of HNE (**IB**), followed by an intra-molecular rearrangement with the formation of the cyclic hemiacetal adduct (**IIB**).

### 1.2 Cellular models

The quenching capacity of H-His-Tyr-OH and H-Tyr-His-OH was subsequently evaluated in a cellular model, using keratinocytes (NCTC 2544 human cell line, Flow Laboratories, Irvine, UK).

Keratinocytes were cultured, at a constant temperature of 37 ± 0.1°C in a humidified 5% CO₂ atmosphere, in DMEM Dulbecco's Modified Eagle's Medium) supplemented with 10% fetal calf serum, 2 mM L-glutamine and antibiotics (100 U/ml penicillin and 0.1 mg/ml streptomycin) until 80-90% confluence as previously described (M. Carini et al., II Farmaco 55, 526-534, 2000)

The experimental model involves the exposure of the cellular mono-layer to the minimal UVB erythematal dose, MED=50 mJ/cm², (Vilber Lourmat VLX-3W radiometer equipped with a UVB probe at 312 nm)and, 4 hours of post-incubation, to the HNE insult (70 nmoles/mg protein).

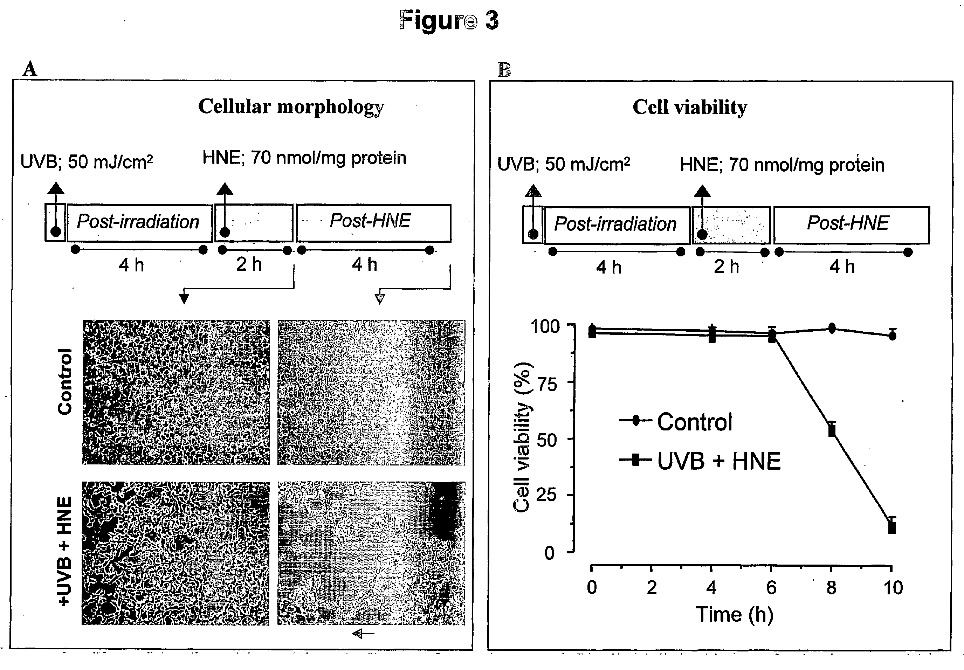

The combination of physical (UVB) and chemical (HNE) stress is used to simulate the conditions of cellular damage of the skin during the cutaneous intrinsic and photo-induced aging process. This stress causes a dramatic morphological alterations of keratinocytcs (phase contrast microscopic examinations, Figure 3, panel A) followed by a significant time-dependent reduction in cell viability, determined according to the calcein assay [Oral H. et al. Endothelium 6, 143-51, 1998] (panel B). In particular, two hours after the addition of HNE (panel c), the keratinocytes show dramatic alterations in cellular morphology (shape, size, etc); after 6 hours, a massive keratinocytes necrosis takes place (panel d), accompanied by an almost complete loss of cell viability (from 95.3 ± 1.2% to 12.3 ± 0.8%; p<0.001).

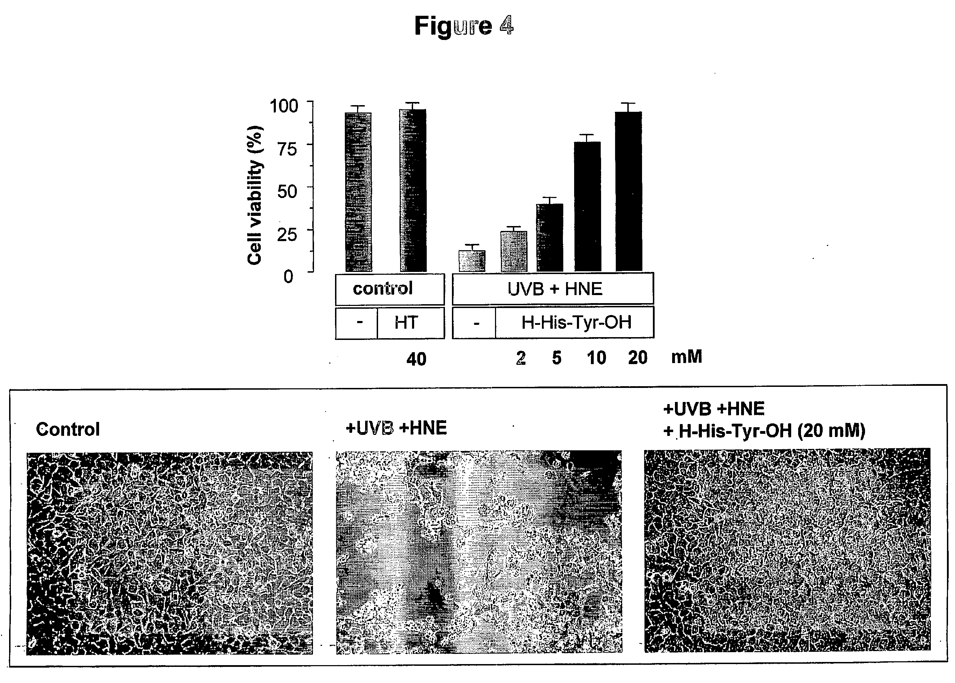

The addition of H-His-Tyr-OH or H-Tyr-His-OH to the incubation medium, concomitant to HNE and UVB exposure, produces a dose-dependent protective effect, as demonstrated by morphological analyses and recovery in cell viability. Figure 4 shows the results relative to the H-His-Tyr-OH dipeptide: the cytoprotective activity (determined 6 hours after the addition of HNE) is already significant at 2 mM and cells are completely protected at 20 mM (morphological analysis, fig. 4).

Mass spectrometric analysis of the extracellular medium evidences the formation of the adduct **IIB**. This demonstrates that the cytoprotective mechanism is due to the ability of the I I-His-Tyr-OII to conjugate and consequently detoxify the cytotoxic agent HNE, thus to prevent its intra-cellular spreading and cytotoxicity. A similar protective effect was observed with the H-Tyr-His-OH compound (data not shown).

### 2. H-His-Tyr-OH and H-Tyr-His-OH antioxidant activity

### 2.1 Cell-free models

The antioxidant activity of H-His-Tyr-OH and H-Tyr-His-OH was demonstrated using the fluorimetric assay of Cao et al. [Free Radio Biol Med, 14(3):303-11, 1993], which deals with the oxidation of the fluorescent substrate R-Phicoerytrine (RPE) by peroxyl radicals generated by the radical initiator AAPH. The amount of RPE oxidation is determined by fluorimetric analysis and the results are expressed as Trolox equivalents (reference compound).

The results (Table II), demonstrate that a 25 µM solution of the dipeptide, object of the present patent, possesses a remarkable anti-oxidant activity (25 µM), which is greater than that of carnosine (the reference dipeptide with a well established antioxidant activity) and of the same order of magnitude in respect to Trolox. (the hydrophilic analogue of vitamin E).

**TABLE II**

| **Substance (25 µM)** | **TROLOX equivalent (µM)** |
|---|---|
| H-His-Tyr-OH | 16.90 ± 0.54 |
| H-Tyr-His-OH | 17.28 ±0.89 |
| Carnosine | 4.94 ± 0.3 |
| Trolox | 25 |

The values are the average ± S.D of 4 determination

### 2.2 Cellular models

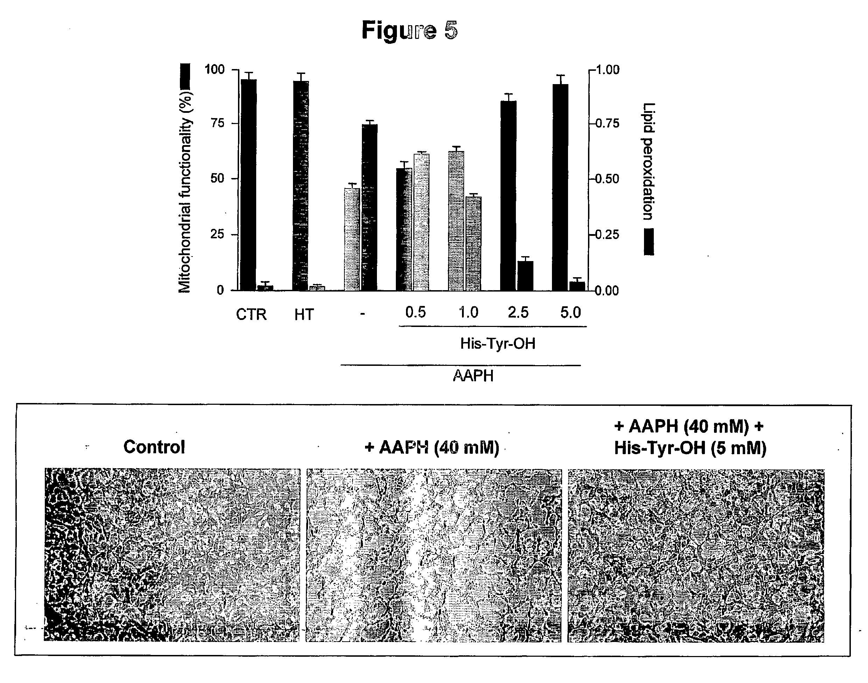

The H-His-Tyr-OH and H-Tyr-His-OH antioxidant activity was subsequently devaluated in a cellular model using human kcratinocytcs cell line (NCTC 2544 human cell line, Flow Laboratories, Irvine, UK), subjected to oxidative damage induced by a flux of peroxyl radicals generated by the radical initiator 2,2'-azobis(2-aminopropane) dihydrochloride (AAPH). The following parameters were evaluated as markers of cellular damage: mitochondrial functionality as an index of cell viability (MTT assay), cellular morphology (phase contrast microscopic examinations), and lipid peroxidation, using 4,4-difluoro-5-(4-phenyl-1,3-butadienyl)-4-bora-3a,4a-diazo-*s*-indacene-3-undecanoic acid(BODIPY 581/591) as membrane fluorescent probe [Pap et al, FEBS Lett. 453:278-282, 1999]. A dramatic cytotoxic effect was observed after 6 hours exposure to 40 mM AAPH(fig.5).

In particular, the morphological examination shows cellular necrosis, concomitant to a reduced mitochondrial functionality (45 ± 3.2 vs 95.6 ± 2.1%; p < 0.001), and to a massive lipid peroxidation process (peroxidation index: 0.743 ± 0.021 vs 0.01 ± 0.005; p < 0.001). The addition of H-His-Tyr-OH to the incubation medium causes a significant and dose-dependent cell protection, which is significant for all the parameters considered, already at 0.5 mM, with a total protection at 10 mM (fig. 5).

### 3. Transition metals chelating ability of H-His-Tyr-OH and H-Tyr-His-OH.

It is known [Chace KV, Carubelli R, Nordquist RE, Rowsey JJ. Free Radic. Res. Commun. 12-13, 591-594, 1991] that transition metals (i.e iron and copper) are able to catalyse free radical-mediated oxidation, according to a Fenton-like mechanism, of easily oxidizable substrates, lipids, proteins, and sugars. These reactions can take place in different sites of human body, as well as in cosmetic and food matrices. The skin (epidermis /derma) represents an anatomical site where transition metals can localize and concentrate in a significant extent following different physicochemical stimulations (i.e UVB stress). A metal-catalyzed oxidative degradation, which involves the basic constituents of the extracellular matrix such as collagen, elastin and jaluronic acid, leads to the onset of accelerated skin aging process.

Hence the possibility of inhibiting the oxidative process catalysed by transition metals, due to a chelating intervention- therefore inactivating the same transition metals - is of great interest for cosmetic, food and pharmaceutical uses.

For this purpose, first we evaluated the ability of H-His-Tyr-OH and H-Tyr-His-OH to chelate copper (II) by spectro-photometric analysis. The peptides are dissolved in phosphate buffer (1 mM) and incubated at 37°C with 1 mM copper sulphate (1:1 molar ratio). Formation of the typical absorption bands of the peptide-copper(II) complex takes place for both H-Tyr-His-OH (λₘₐₓ=600 nm) and for H-His-Tyr-OH (λₘₐₓ=650 nm), accompanied by the concomitant disappearance of the typical absorption band of free copper (II) at 800 nm.

Since at subcutaneous level transition metals can interact with collagen and induce protein destructuration, in a second model system we evaluated the copper-sequestering ability of H-His-Tyr-OH and H-Tyr-His-OH in a medium containing 1 mg/ml Type III collagen suspended in 1 mM phosphate buffer (pH 7.4). The interaction copper (II) - collagen was demonstrated by monitoring the disappearance of the absorption band typical of copper (II).

Addition of the dipeptides to the reaction mixtures results in the appearance of the absorption bands typical of peptide-copper (II) complex previously described, as a demonstration of the capacity of these dipeptides to sequester copper ions from collagen, making it less susceptible to the oxidative degradation catalyzed by copper (II), and more resistant to stress and photo-aging.

### 4. Functionalized dipetides

The second important object of the present invention deals with the design/discovery of new compounds obtained by suitable functionalization of dipeptides **A.** and **B.,** which arc equipotent as antioxidant/quencher to the parent molecules.

Functionalization can involve the amine and/or the hydroxyl and/or the carboxyl groups of **A.** and **B.** positions, The compounds deriving therefrom, object of the present invention, can be described by the following general formulae, with the understanding that one or more substitutions can be present in the same molecule, and the relevant compounds can be used as inhibitors or preventive agents of degradation phenomena, as such or as a mixture of two or more or them, or in suitable formulations. 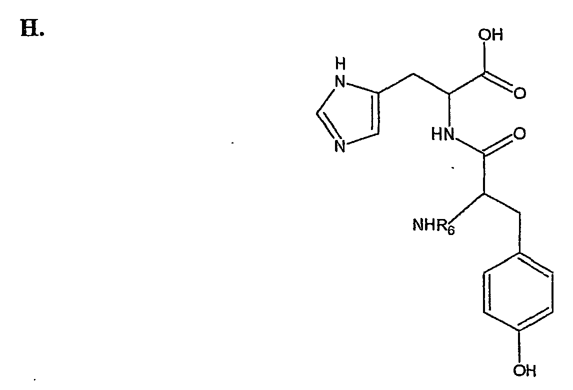 wherein the substituent R₆ can be:
- -CO(CH₂)nCH₃ with n ranging from 0 to 18, linear, branched, saturated or unsaturated
   For illustrative purposes, some applicative examples of the previously defined compounds for cosmetic, food and pharmaceutical uses are below reported:
- inhibition of the oxidative processes of raw materials, intermediate and finished products for cosmetic food, and pharmaceutical uses;
- inhibition of the oxidative damage of physio-pathological origin of human and animal body tissues, following administration of the compounds defined in claims 3 and 4, by oral, by topical, rectal or parent -eral administration.
- inhibition/prevention of the degradation/degeneration processes induced by the oxidative-driven aldehydes/keto-aldehydes in raw materials, intermediate and finished products for cosmetic, food and pharmaceutical uses;
- inhibition/prevention of the degradation/degeneration processes induced by the oxidative-driven aldehydes/keto-aldehydes in different areas/compartments of human and animal body, following administration of the compounds defined in claims 3 and 4, by oral, topical, rectal or parenteral administration.

The compounds described in the formulae from C to **H** can be easily obtained according to methods that are well-known to any expert in the field who will select the most suitable method of synthesis on the basis of the desired functionalization reaction. For example, the methods described in the following textbooks may be applied:000
- J. March: Advanced Organic Chemistry, 4th Edition, Wiley Interscience (1992);
- M. Bodanszky: Principles of peptide synthesis. Ed Springer Laboratory (1993);
- W.C. Chan, P.D. White: Fmoc Solid phase peptide synthesis: a practical approach. Ed. Peter White Editor, IRL press. (2000)

For illustrative purposes, a brief descriptive scheme of the synthesis is provided below, together with the analytical characterization of the N-palmitoyl derivative from H-Tyr-His-OH (2-[2-hexadecanoylamino-3-(4-hydroxy phenyl)-propionylamino]3-(3H-4-yl)-propionic acid), whose structure is shown below 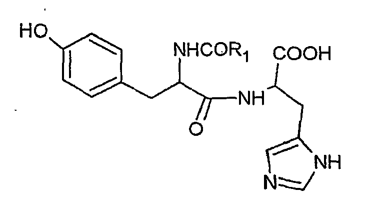 wherein R₁ is an acyl hexadecanoyl chain.

The reaction scheme (figure VI) involves the preparation of 3-(4-tert-butoxyphenyl)-2-hexadecanoyl amino propionic acid (**II**) by reaction of 2-amino-3-(4-tert-butoxyphenyl) propionic acid (**I**) with palmitoyl chloride; functionalization of the carboxylic residue with N-hydroxysuccinimide to give compound **III**; formation of the carboamide **V** derivative by reaction of compound **III** with the iso-butyl ester of L-histidine (**IV**); de-protection of the carboxylic function by alkaline hydrolysis (**VI**), subsequent de-protection of the phenolic function (acid hydrolysis by treatment with trifluoroacetic acid; TFA) to give 2-[2-hexadecanoylamino-3-(4-hydroxyphenyl)-propionyl amino]3-(3H-4-yl)-propionic acid (**VII**).

The structure confirmation of **VII** was established by 1H-NMR, IR and ESI-MS analyses as indicated below: 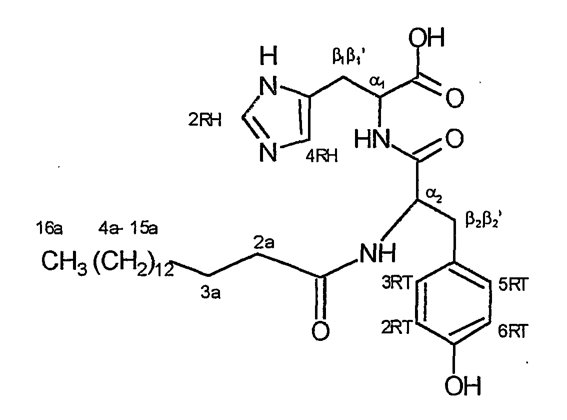 *1H-NMR* (DMSO, 300 MHz): 2RH (δ 8.3,s, 1H); 4RH (δ 7.1,s, 1H); β₁β₁'(δ 2.9, m, 2H); α₁(δ 2.9, m, 2H); α₂ (δ 2.9, m, 1H); β₂β₂'(δ 2.9, m, 2H); 3,5 RT (δ 6.6, D, 2H); 2,6 RT (δ 6.6, D, 2H); 2a (δ 2.3, t, 2H); 3a (δ 1.48, t, 2H); 4a-15a (δ 1.31, m, 12H); 16a (δ 0.9, t, 3H) *IR (cm⁻¹)(KBr):* 3500-3200 (N-H, O-H); 3000-2840 (C-H aliphatic); 1750 (C=O ester); 1650 (C=O amide); 1595, 1500 (C=C aromatic); 1250 (C-O).
*ESI-MS_{:}* positive: m/z 557.8 [M+H]⁺; negative: m/z 555.8 [M-H]⁻
All the above compounds can be used as such, or incorporated into suitable formulations, whose physical state (liquid, solid, creamy, spray, etc) and type of carrier (capsule, pills, tablets, etc) is selected, once again, by experts in the field, according to the specific utilization, both in the cosmetic, food and pharmaceutical fields. The composition, also object of the present invention, will include, as active molecule, one or more of the compounds according to the above-mentioned formulations, and also at least one of the following products: surface-active agents, emulsifiers, adhesives, preservatives, gel agents, water, solvents, inorganic excipients, lubricants, binders and stabilizers.
In addition the dipeptides of the present invention can be incorporated directly and without further modification in foods, nutraceuticals, beverages by conventional techniques such as mixing, infusion, injection, blending, dispersing, emulsifying, immersion, spraying and kneading.
It is contemplated within the scope of the present invention that the di-peptides and the functionalized derivatives object of the present invention may be incorporated into various conventional pharmaceutical preparations, and dosage form, such as tablets, (plain and coated) for use orally, bucally or lingually, capsules (hard and soft, gelatin, with or without additional coatings, powders, granules (including effervescent granules), pellets, microparticulates, solutions (such as micellar, syrups, elixirs and drops) lozenges, pastilles, ampoules, emulsions, microemulsions, ointments, creams, suppositories, gels, transdermal patches, and modified release dosage forms together with customary excipients and/or diluents and stabilizers.

Some examples of formulation for skin and hair protection are indicated hereunder, for a better understanding of the details of the present invention, whose object should not be considered limited to these examples.

### FORMULATION EXAMPLES

Physico-chemical forms
- 1.: Solutions
- 2.: Surfactants containing suspensions
- 3.: Gels
- 4.: Aqueous pastes
- 5.: Gel emulsions
- 6.: O/A emulsions
- 7.: A/O emulsions
- 8.: Anhydrous lipidic systems

Formulation examples in which the active principle was inserted at 0,5% in all formulations, even if this percentage can be increased, without destabilizing the formulate.

The active principle is represented by the following abbreviation: HIS-TYR

### SOLUTIONS:

(a) NON ALCOHOLIC AQUEOUS
(b) HYDROALCOHOLIC
(c) SILICONE-ALCOHOL
(d) OILY

### Example a: FACE TONIC

| | | |
|---|---|---|
| 1. | PRESERVED WATER | q.s. to 100% |
| 2. | GLYCERIN | 5 |
| 3. | PEG-40 HYDROGENATED CASTOR OIL | 1 |
| 4. | PARFUM | 0.2 |
| 5. | HIS-TYR | 0.5 |
| 6. | CITRIC ACID | q.s. to pH = 5.5 |

### Example b: HYDROALCOHOLIC AFTER SHAVE LOTION

| | | |
|---|---|---|
| 1. | PRESERVED WATER | q. s. to 100% |
| 2. | DENAT. ALCOHOL | 20 |
| 3. | CITRIC ACID | 0.2 |
| 4. | PANTHENOL | 1 |
| 5. | HIS-TYR | 0.5 |
| 6. | PEG-40 HYDROGENATED CASTOR OIL | 1 |
| 7. | PARFUM | 1 |

### Example c: DEODORANT LOTION

| | | |
|---|---|---|
| 1 | DENAT. ALCOHOL | 68.5 |
| 2 | PARFUM | 1 |
| 3 | HIS-TYR | 0.5 |
| 4 | CYCLOPENTASILOXANE | 30 |

### Example d: BABY OIL

| | | |
|---|---|---|
| 1. | SQUALENE | 30 |
| 2. | CALENDULA OFFICINALIS | 10 |
| 3. | AVOCADO OIL | 10 |
| 4. | CAPRYLIC/CAPRIC TRIGLYCERIDE | 49 |
| 5. | HIS-TYR | 0.5 |
| 6. | PARFUM | 0.5 |

### DETERGENTS:

| | |
|---|---|
| (a) | SHAMPOO |
| (b) | SHAMPOO AND SHOWER BATH |
| (c) | BATH OIL |
| (d) | FACE AND HAND SOAP |
| (e) | SOFT BODY SOAP |
| (f) | FOOT BATHS |

### Example a: SHAMPOO

| | | |
|---|---|---|
| 1. | SODIUM LAURETH SULFATE (27%) | 30 |
| 2. | SODIUM COCOAMPHOACETATE (30%) | 5 |
| 3. | COCAMIDOPROPYL BETAINE | 5 |
| 4. | PRESERVED WATER | q.s. to 100% |
| 5. | SODIUM CHLORIDE | q.s. |
| 6. | PARFUM | 0.5 |
| 7. | HIS-TYR | 0.5 |

### Example b: SHAMPOO AND SHOWER BATH

| | | |
|---|---|---|
| 1. | SODIUM LAURETH SULFATE (27%) | 35 |
| 2. | SODIUM COCOAMPHOACETATE (30%) | 15 |
| 3. | CAPRYLIL/CAPRYL GLUCOSIDE (60%) | 5 |
| 4. | COCAMIDOPROPYL BETAINE (30%) | 8 |
| 5. | PRESERVED WATER | q.s. to 100% |
| 6. | PARFUM | 1 |
| 7. | SODIUM CHLORIDE | 1 |
| 8. | HIS-TYR | 0.5 |
| 9. | CITRIC ACID | q. s. to pH= 5.5 |

### Example c: BATH OIL

| | | |
|---|---|---|
| 1. | POLYGLYCERYL-6 ISOSTEARATE | 12.5 |
| 2. | ISOSTEARYL ISOSTEARATE | 8 |
| 3. | PARFUM | 2 |
| 4. | PRESERVED WATER | 50 |
| 5. | PEG-7 GLYCERYL COCOATE | 27 |
| 6. | HIS-TYR | 0.5 |

### Example d: FACE AND HAND SOAP NO GAS FOAM MOUSSE

| | | |
|---|---|---|
| 1. | SODIUM LAURETH SULFATE (27%) | 2.5 |
| 2. | COCAMIDOPROPYL BETAINE (30%) | 2.5 |
| 3. | SODIUM LAUROYL SARCOSINATE | 2.5 |
| 4. | QUILLAIA SAPONARIA | 5 |
| 5. | GLYCERIN | 5 |
| 6. | HYDROXYPROPYL GUAR | 0.1 |
| 7. | HIS-TYR | 0.5 |
| 8. | PARFUM | 0.2 |
| 9. | PRESERVED WATER | q.s. to 100% |
| 10. | CITRIC ACID | q.s. to pH= 5.5 |

### Example e: SOFT BODY SOAP

| | | |
|---|---|---|
| 1. | PRESERVED WATER | q.s. to 100% |
| 2. | HYDROXYPROPYL GUAR | 1.5 |
| 3. | SODIUM LAUROYL SARCOSINATE | 10 |
| 4. | COCAMIDOPROPYL BETAINE (30%) | 10 |
| 5. | PARFUM | 0.5 |
| 6. | LACTIC ACID | q.s. to pH = 4 |
| 7. | HIS-TYR | 0.5 |
| 8. | PEG-120 METHYL GLUCOSE DIOLEATE | 2 |

### Example f: FOOT BATH

| | | |
|---|---|---|
| 1. | PRESERVED WATER | q.s. to 100% |
| 2. | ZINC COCETH SULFATE | 30 |
| 3. | DISODIUM COCOAMPHODIACETATE | 10 |
| 4. | PARFUM | 1 |
| 5. | SODIUM CHLORIDE | 1 |
| 6. | CITRIC ACID | q.s. to pH=4 |
| 7. | HIS-TYR | 0.5 |

### GELS

### Example: MOISTURIZING GEL

| | |
|---|---|
| 1. PRESERVED WATER | q.s. to 100% |
| 2. CARBOMER | 0.5 |
| 3. SODIUM HYDROXIDE 1N. | 0.5 |
| 4. POLYSORBATE 20 | 0.5 |
| 5. PARFUM | 0.1 |
| 6. HIS-TYR | 0.5 |
| 7. SACCHARIDE ISOMERATE AQUEOUS PASTES | 2 |

### Example : CLAY MASK

| 1. | PRESERVED WATER | q.s. to 100% |
|---|---|---|
| 2. | HECTORITE, HYDROXYETHYLCELLULOSE | 1.75 |
| 3. | HYDROXYETHYLCELLULOSE | 1 |
| 4. | CALCIUM CARBONATE | 8 |
| 5. | KAOLIN | 10 |
| 6. | SILICA | 2.5 |
| 7. | PARFUM | 0.5 |
| 8. | GLYCERIN | 4 |
| 9. | PEG-7 GLYCERYL COCOATE | 2.5 |
| 10. | HIS-TYR | 0.5 |
| 11. | COCAMIDOPROPYL BETAINE | 2.5 |

### Example: TOOTH PASTE

| | | |
|---|---|---|
| 1. | PRESERVED WATER | q.s. to 100% |
| 2. | CELLULOSE GUM | 0.5 |
| 3. | SORBITOL | 4 |
| 4. | XYLITOL | 2 |
| 5. | GLYCERIN | 15 |
| 6. | SILICA | 50 |
| 7. | SODIUM LAUROYL SARCOSINATE (30%) | 4 |
| 8. | AROMA | 1 |
| 9. | POLYSORBATE 20 | 1 |
| 10. | HIS-TYR | 0.5 |

### GEL EMULSIONS

### Example: CLEANSING MILK

| | | |
|---|---|---|
| 1. | PRESERVED WATER | q.s. to 100% |
| 2. | ACRYLATES/C10-30 ALKYL ACRYLATE CROSSPOLYMER | 0.2 |
| 3. | CARBOMER | 0.1 |
| 4. | AMINOMETHYL PROPANOL | 0.25 |
| 5. | ETHYLHEXYL ETHYLHEXANOATE | 5 |
| 6. | C12-15 ALKYL BENZOATE | 5 |
| 7. | DIMETHICONE | 1 |
| 8. | HIS-TYR | 0.5 |
| 9. | PARFUM | 0.25 |

### Example : GEL SUN CREAM

| | | |
|---|---|---|
| 1. | ETHYLHEXYL METHOXYCINNAMATE | 5 |
| 2. | TITANIUM DIOXIDE | 3 |
| 3. | ETHYLHEXYL ETHYLHEXANOATE | 5 |
| 4. | BUTYROSPERMUM PARKII | 2 |
| 5. | TOCOPHERYL ACETATE | 1 |
| 6. | CARBOMER | 0.25 |
| 7. | ACRYLATES/C10-30 ALKYL ACRYLATE CROSSPOLYMER | 0.15 |
| 8. | AMINOMETHYL PROPANOL | 0.3 |
| 9. | GLYCERIN | 3 |
| 10. | PARFUM | 0.5 |
| 11. | PRESERVED WATER | q.s. to 100% |
| 12. | HIS-TYR | 0.5 |

### O/A EMULSIONS

### Example: HAIR BALSAM

| | | |
|---|---|---|
| 1. | PRESERVED WATER | q.s. to 100% |
| 2. | CETEARYL ALCOHOL | 5 |
| 3. | CETRIMONIUM CHLORIDE | 2 |
| 4. | QUATERNIUM-80 | 1 |
| 5. | CETEARETH-20 | 1.5 |
| 6. | OCTYLDODECANOL | 5 |
| 7. | PROPYLENE GLYCOL | 2 |
| 8. | HYDROXYPROPYL GUAR | 0.3 |
| 9. | HIS-TYR | 0.5 |
| 10. | PARFUM | 0.3 |
| 11. | CITRIC ACID | 0.2 |

### Example : BODY EMULSIONS

| | | |
|---|---|---|
| 1. | POTASSIUM PALMITOYL HYDROLYZED SWEET ALMOND PROTEIN, GLYCERYL STEARATE,CETARYL ALCOHOL, SOY STEROL | 5 % |
| 2. | ETHYLHEXYL METHOXYCINNAMATE | 2 |
| 3. | CAPRYLIC/CAPRIC TRIGLYCERIDE | 3 |
| 4. | MACADAMIA TERNIFOLIA | 3 |
| 5. | ETHYLHEXYLETHYLHEXANOATE | 10 |
| 6. | DIMETHICONE | 0.5 |
| 7. | PRESERVED WATER | q.s. to 100 % |
| 8. | GLYCERIN | 0.25 |
| 9. | HIS-TYR | 0.5 |
| 10. | PARFUM | 0.25 |

### Example: FACE CREAM

| | |
|---|---|
| 1. CETEARYL GLUCOSIDE, CETEARYL ALCOHOL 5 | |
| 2. GLYCERYL STEARATE | 1,5 |
| 3. DECYL OLEATE | 2 |
| 4. ETHYLHEXYL ETHYLHEXANOATE | 4 |
| 5. ORYZANOL | 1 |
| 6. PRUNUS DULCIS | 5 |
| 7. TOCOPHERYL ACETATE | 1 |
| 8. DIMETHICONE | 0.5 |
| 9. OLEAEUROPAEA | 3 |
| 10. PRESERVED WATER | q.s. to 100 % |
| 11. GLYCERIN | 3 |
| 12. HIS-TYR | 0.5 |
| 13. PARFUM | 0.25 |

### A/O EMULSIONS

| | | |
|---|---|---|
| 1. | POLYGLYCERYL-2 DIPOLYHYDROXYSTEARATE | 3 |
| 2. | GLYCERYL OLEATE | 1 |
| 3. | CAPRYLIC/CAPRYC TRIGLYCERIDE | 5 |
| 4. | C12-15 ALKYL BENZOATE | 5 |
| 5. | POLYDECENE | 7 |
| 6. | PRESERVED WATER | q.s. to 100 % |
| 7. | MAGNESIUM SULFATE | 0.5 |
| 8. | HIS-TYR | 0.5 |
| 9. | GLYCERIN | 3 |
| 10. PARFUM | | 0.5 |

### ANHYDROUS LIPIDIC SYSTEMS

### Example : LIPSTICK

| | | |
|---|---|---|
| 1. | CERA ALBA | 8.5 |
| 2. | CARNAUBA WAX | 3.5 |
| 3. | LANOLINE | 3.5 |
| 4. | ISOPROPYLPALMITATE | 7.5 |
| 5. | AROMA | 0.5 |
| 6. | TOCOPHEROL | 0.1 |
| 7. | PROPYLPARABEN | 0.1 |
| 8. | CASTOR OlL | q.s. to 100 % |
| 9. | MICA | 10.5 |
| 10. | HIS-TYR | 0.5 |
| 11. | PIGMENTS | 4.5 |

## Claims

1. Use of peptides selected from H-His-Tyr-OH and/or H-Tyr-His-OH and/or N-acyl derivatives of the Tyr part of H-Tyr-His-OH, said acyl group having the formula -CO-(-CH₂)ₙ-CH₃ wherein n is from 0 to 18, for the manufacture of a medicament or a cosmetic product for the inhibition/prevention of the oxidative damage of the skin.

2. Use according to claim 1, wherein the damage of the skin is due to intrinsic- or photo-aging of the skin.

3. Use according to claim 1, wherein the damage of the skin is due to physical (UV, vis IR radiations), thermal and mechanical stress of the skin.

4. Use according to claim 1, wherein the medicament or the cosmetic product is formulated as solution, surfactants containing suspension, gel, aqueous paste, gel emulsion, o/w emulsion, w/o emulsion, anhydrous lipidic system.

5. N-acyl derivatives of the Tyr part of H-Tyr-His-OH, said acyl group having the formula - CO-(-CH₂)ₙ-CH₃ wherein n is from 0 to 18.

6. N-acyl compound according to claim 5, wherein n = 14

## Patentansprüche

1. Verwendung von Dipeptiden ausgewählt aus H-His-Tyr-OH und/oder H-Tyr-His-OH und/oder N-Acyl-Derivaten des Tyr-Teils von H-Tyr-His-OH, wobei die Acylgruppe die Formel -CO-(-CH₂)ₙ-CH₃, worin n von 0 bis 18 ist, aufweist, zur Herstellung eines Medikaments oder eines Kosmetikprodukts zur Inhibierung/Verhinderung der oxidativen Schädigung der Haut.

2. Verwendung nach Anspruch 1, wobei die Schädigung der Haut auf intrinsisches Altern oder Lichtalterung zurückzuführen ist.

3. Verwendung nach Anspruch 1, wobei die Schädigung der Haut auf eine physikalische (UV-,VIS-, IR-Strahlung), thermische und mechanische Beanspruchung der Haut zurückzuführen ist.

4. Verwendung nach Anspruch 1, wobei das Medikament oder das Kosmetikprodukt formuliert ist als Lösung, tensidhaltige Suspension, Gel, wässrige Paste, Gel-Emulsion, O/W Emulsion, W/O-Emulsion, wasserfreies Lipidsystem.

5. N-Acyl-Derivate des Tyr-Teils von H-Tyr-His-OH, wobei die Acylgruppe die Formel -CO-(CH₂)ₙ-CH₃ aufweist, worin n von 0 bis 18 ist.

6. N-Acyl-Verbindung nach Anspruch 5, worin n =14.

## Revendications

1. Utilisation de dipeptides choisis parmi le H-His-Tyr-OH et/ou le H-Tyr-His-OH et/ou les dérivés N-acyles de la partie Tyr de H-Tyr-His-OH, ledit groupe acyle ayant la formule suivante :
-CO-(-CH₂)ₙ-CH₃
dans lequel n est compris entre 0 et 18,
pour la fabrication d'un médicament ou d'un produit cosmétique pour l'inhibition ou la prévention des lésions oxydatives de la peau.

2. Utilisation selon la revendication 1, dans laquelle les lésions de la peau sont dues au vieillissement intrinsèque ou au photo-vieillissement de la peau.

3. Utilisation selon la revendication 1, dans laquelle les lésions de la peau sont dues à une sollicitation physique (rayonnements UV, IR visible), thermique ou mécanique de la peau.

4. Utilisation selon la revendication 1, dans laquelle la formulation du médicament ou du produit cosmétique se présente sous la forme d'une solution, d'une suspension contenant des surfactants, d'un gel, d'une pâte aqueuse, d'une émulsion en gel, d'une émulsion d'huile dans l'eau, d'une émulsion d'eau dans l'huile ou d'un système lipidique anhydre.

5. Dérivés N-acyles de la partie Tyr de H-Tyr-His-OH, ledit groupe acyle ayant la formule suivante :
-CO-(-CH₂)n-CH₃
dans lequel n est compris entre 0 et 18.

6. Composé N-acyle selon la revendication 5, dans lequel n = 14.
